**Europäisches Patentamt**

**European Patent Office**

**Office européen des brevets**

(19)

(11) Numéro de publication : **0 390 653 B1**

(12) **FASCICULE DE BREVET EUROPEEN**

(45) Date de publication du fascicule du brevet :
**17.06.92 Bulletin 92/25**

(21) Numéro de dépôt : **90400790.3**

(22) Date de dépôt : **22.03.90**

(51) Int. Cl.⁵ : **A61B 6/00**

(54) **Mammographe équipé d'un dispositif de prises de vues stéréotaxiques intégré et procédé d'utilisation d'un tel mammographe.**

(30) Priorité : **29.03.89 FR 8904086**

(43) Date de publication de la demande :
**03.10.90 Bulletin 90/40**

(45) Mention de la délivrance du brevet :
**17.06.92 Bulletin 92/25**

(84) Etats contractants désignés :
**DE ES GB IT NL**

(56) Documents cités :
**EP-A- 0 071 017**
**EP-A- 0 127 073**
**EP-A- 0 146 511**
**EP-A- 0 288 187**
**FR-A- 1 541 671**

(73) Titulaire : **GENERAL ELECTRIC CGR S.A.**
**100, rue Camille-Desmoulins**
**F-92130 Issy les Moulineaux (FR)**

(72) Inventeur : **Romeas, René, Cabinet Ballot-Schmit**
**7, rue Le Sueur**
**F-75116 Paris (FR)**
Inventeur : **Rouchy, Didier, Cabinet Ballot-Schmit**
**7, rue Le Sueur**
**F-75116 Paris (FR)**
Inventeur : **Gilleron, Alain, Cabinet Ballot-Schmit**
**7, rue Le Sueur**
**F-75116 Paris (FR)**

(74) Mandataire : **Schmit, Christian Norbert Marie et al**
**Cabinet Ballot-Schmit 7, rue Le Sueur**
**F-75116 Paris (FR)**

## Description

La présente invention a pour objet un mammographe équipé d'un dispositif de prise de vues stéréotaxiques intégré ainsi qu'un procédé d'utilisation d'un tel mammographe. L'invention s'applique principalement au domaine médical où la mammographie apparaît comme étant le meilleur moyen de détection préventive ou de traitement des cancers du sein. Les prises de vue stéréotaxiques sont des prises de vue permettant de déterminer par calcul la position d'une tumeur ou d'une lésion dans un sein examiné.

Un appareil de mammographie se compose principalement d'un bras mobile autour d'un axe portant à une de ses extrémités un tube générateur de rayons X, et à une autre extrémité, un récepteur d'images. Le plus souvent le récepteur d'images comporte une cassette munie d'un film et d'un écran renforçateur d'images. Sur le trajet du faisceau des rayonnements X émis par le tube sont intercalés différents dispositifs. On trouve normalement, en amont à proximité du tube, un limiteur de faisceau. Plus en aval, et à proximité d'un plateau porte-sein on trouve une pelote ou plateau de compression du sein. Ce plateau porte-sein et ce plateau de compression sont destinés à maintenir le sein en place au moment des prises de vues radiographiques. Ils sont normalement solidaires du tube et du récepteur. Mais ils peuvent en être désolidarisés. Le fait que le tube et le récepteur d'images soient mobiles en rotation, éventuellement autour du plateau porte-sein et du plateau de compression, ou solidairement avec eux, peut permettre de réaliser des prises de vues stéréotaxiques.

Pour réaliser ces prises de vues stéréotaxiques, on procède de la manière suivante. On utilise un deuxième récepteur d'image qu'on fixe près du plateau porte-sein. On incline le tube à rayons X, solidaire du premier récepteur d'images, selon une première orientation par rapport à l'alignement du plateau porte-sein et du plateau de compression, on introduit une cassette dans le deuxième récepteur d'images et on prend un premier cliché. La cassette est pour le premier cliché décalée latéralement d'un côté dans le deuxième récepteur d'images, de sorte que seulement une première partie latérale du film qu'elle contient est impressionnée par la première exposition. On décale ensuite la cassette de l'autre côté dans le récepteur d'images, on incline le tube à rayons X selon une seconde orientation, et on prend un deuxième cliché. Ultérieurement on développe le film correspondant aux deux expositions et, au moyen d'un dispositif de localisation stéréotaxique, qui comporte dans son principe de fonctionnement le traitement par ordinateur des deux images obtenues, on détermine la position dans l'espace d'une tumeur éventuelle, puis on guide avec précision une aiguille de biopsie à des fins thérapeutique.

Le document EP-A-0 146 511 décrit un mammographe selon le préambule de la revendication 1. Ce mammographe permet la mise en oeuvre de la méthode décrite ci-dessus.

L'examen de mammographie est un examen relativement pénible pour la patiente. En conséquence il importe d'agir rapidement. En effet, les dispositifs ainsi décrits de l'état de la technique présentent l'inconvénient au contraire d'être lents du fait qu'il faut décaler la cassette entre les expositions, développer le film, calculer la position, guider l'aiguille, avant de pouvoir relâcher la compression sur le sein. D'une part les appareils connus sont ainsi peu maniables, d'autre part ils sont traumatisants pour les patientes.

Cependant, ces appareils connus ont des fonctionnalités multiples. Notamment ils doivent permettre de prendre des prises de vues du sein selon différentes incidences en particulier verticales. Il importe donc qu'un matériel qui résoudrait les problèmes de manipulation évoqués ne le fasse pas au détriment des possibilités d'utilisation des appareils de type connu.

Quand on utilise une cassette porte film plus grande et qu'on la déplace entre les deux clichés, on ne superpose pas les deux prises de vue selon les deux incidences. Cependant cette technique nécessite d'une part de disposer d'une référence de position zéro sur chacun des clichés, et elle entraîne d'autre part un agrandissement du porte-cassette dans le récepteur d'images. En effet celui-ci doit avoir une largeur égale à la longueur de la cassette plus la longueur du déplacement nécessaire. Ceci peut causer une gêne lors de la mise en place de la patiente, en particulier en incidence oblique.

L'invention a pour objet de remédier à ces inconvénients en proposant deux organes mobiles pour supporter les accessoires du mammographe. Ces deux organes sont mobiles d'une part par rapport au support général du mammographe, et mobile d'autre part l'un par rapport à l'autre. Un de ces organes mobiles porte le tube à rayons X, l'autre porte le reste des accessoires : c'est-à-dire, le plateau de compression, le plateau porte-sein et l'unique récepteur d'images. Le récepteur d'image est ainsi désolidarisé du tube à rayons X. Pour les prises de vues stéréotaxiques, on écarte de plus le récepteur d'images et on bénéficie alors d'un effet de parallaxe qui résulte du décalage en projection de l'image projetée sur un récepteur fixe d'images, d'un objet fixe, quand le centre de projection a bougé. On choisit alors simplement de faire bouger la position du tube à rayons X d'un angle suffisant pour que les deux images projetées du sein radiographié soient juxtaposées l'une à l'autre. Pour rendre cette séparation des images d'autant plus efficace, on choisit d'écarter le récepteur d'images du sein radiographié d'une distance qui est fonction de la variation d'inclinaison du tube à rayons X.

L'invention a donc pour objet un mammographe comportant des moyens pour maintenir en regard d'un sein à radiographier un tube à rayons X d'une part, et un plateau porte-sein et un récepteur d'images radiographiques d'autre part, les moyens comportant deux bras,

    – un premier bras mobile en rotation autour d'un premier axe perpendiculaire à ce premier bras, ce premier bras portant le tube à rayons X pour orienter le rayonnement de ce tube,

    – un deuxième bras portant le plateau porte-sein et le récepteur d'images, caractérisé en ce que le deuxième bras est mobile en rotation autour d'un deuxième axe perpendiculaire à ce deuxième bras et dans un plan parallèle au plan de rotation du premier bras

et en ce que le deuxième bras comportant des moyens pour ménager un espace variable entre le plateau porte-sein et le récepteur d'images pour qu'une première image du sein projetée sur le récepteur d'images et correspondant à une première orientation du rayonnement du tube, soit juxtaposée sur ce récepteur d'images à une deuxième autre image du même sein, projetée dans les mêmes conditions, mais correspondant à une deuxième autre orientation du rayonnement de ce tube, ces orientations étant obtenues pour des positions différentes du premier bras par rapport au deuxième bras.

L'invention a également pour but un procédé de prises de vues stéréotaxiques d'un sein avec un mammographe, dans lequel on effectue les étapes suivantes :

    – on place un sein à radiographier entre un tube à rayons X et un plateau porte-sein,

    – on place un récepteur d'images muni d'un film radiosensible en aval de ce plateau porte-sein,

    – on écarte ce récepteur d'images de ce plateau porte-sein, et on le rend solidaire de ce plateau porte-sein,

    – on incline le tube à rayons X d'un côté de la normale au plateau porte-sein, en maintenant immobile ce plateau porte-sein et ce récepteur d'images,

    – on prend un cliché,

    – on incline le tube d'un autre côté par rapport à cette normale sans modifier ni la position du film radiosensible ni le récepteur d'images,

    – on prend un deuxième cliché,

    – et on développe l'image globale ainsi obtenue, projetée en deux images juxtaposées sur un film radiosensible unique introduit dans le récepteur d'images.

L'invention sera mieux comprise à la lecture de la description qui suit et à l'examen des figures qui l'accompagnent. Celles-ci ne sont données qu'à titre indicatif et nullement limitatif de l'invention. Les figures montrent :

    – figure 1 : une représentation schématique en coupe d'un mammographe selon l'invention.

    – figure 2 : une représentation schématique d'une vue de face du mammographe selon l'invention, pendant une prise de vue stéréotaxique.

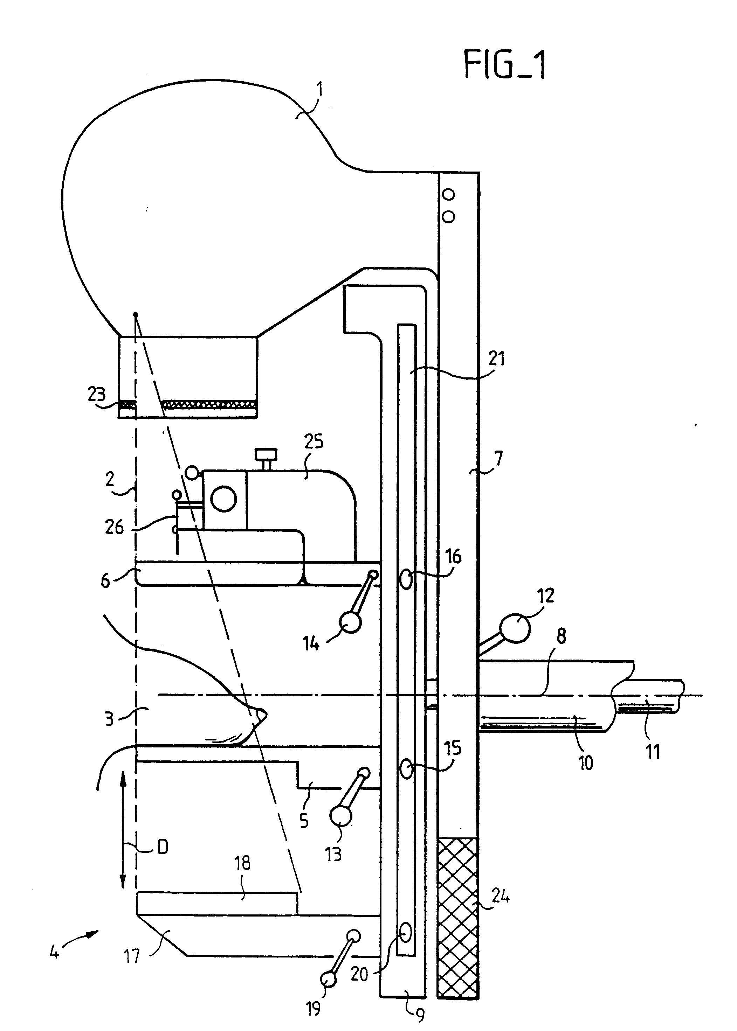

La figure 1 montre un mammographe selon l'invention. Celui-ci comporte un tube générateur de rayons X 1 émettant un rayonnement X 2 en direction d'un sein 3 à radiographier et d'un récepteur d'images 4. Le sein 3 à radiographier est porté par un plateau 5 porte-sein. Il peut être comprimé par un plateau de compression 6. Le dispositif de l'invention comporte un premier bras 7 mobile en rotation autour d'un premier axe 8 perpendiculaire à ce premier bras. L'axe 8 est sensiblement horizontal. Le premier bras 7 porte à son extrémité, fixé par tous moyens, le générateur de rayons X 1. Un deuxième bras 9 est également mobile en rotation autour d'un axe perpendiculaire à ce deuxième bras. De manière préférée l'axe de rotation du deuxième bras est confondu avec l'axe 8 de rotation du premier bras 7. Il pourrait cependant en être autrement. Dans cette variante préférée cette confusion des axes est obtenue en réalisant, pour la rotation du premier bras 7, un arbre creux 10. Dans cet arbre creux un arbre intérieur peut tourner en rotation exactement. L'arbre 11 fixé au deuxième bras 9 autorise la rotation de ce deuxième bras 9. Pour certaines utilisations envisagées plus loin, ces deux arbres peuvent être rendus solidaires, dans n'importe quelle position de l'un par rapport à l'autre, au moyen d'un frein non représenté actionné par une poignée de frein 12. Ce frein, fixé par exemple a l'arbre 10, vient s'appuyer quand on manoeuvre la poignée 12 sur l'arbre 11 et empêche ce dernier de continuer à tourner librement par rapport à l'arbre 10.

D'une manière connue le plateau porte-sein 5 et le plateau de compression 6 peuvent être réglés en hauteur, d'une part, l'un par rapport à l'autre, et d'autre part par rapport à l'axe 8 de rotation du premier bras 7. Une particularité de l'invention réside dans le fait que des poignées 13 et 14, respectivement de réglage des hauteurs du plateau porte-sein 5 et du plateau de compression 6 agissent sur des verrous 15, 16 de blocage de leur position respective sur le deuxième bras 9. Le récepteur d'images 4 comporte un plateau 17 agencé pour recevoir une cassette 18. Ce plateau 17 peut être réglé en hauteur par une poignée 19 agissant sur un verrou 20. Ce verrou 20 maintient le plateau 17 par rapport au deuxième bras 9. Les verrous 15, 16 et 20 peuvent avoir toutes formes. Dans un exemple ils peuvent comporter une excentrique comprimable qui vient s'engager à force, dans un logement 21 réalisé dans le deuxième bras 9, quand on tourne les poignées 13, 14, et 19.

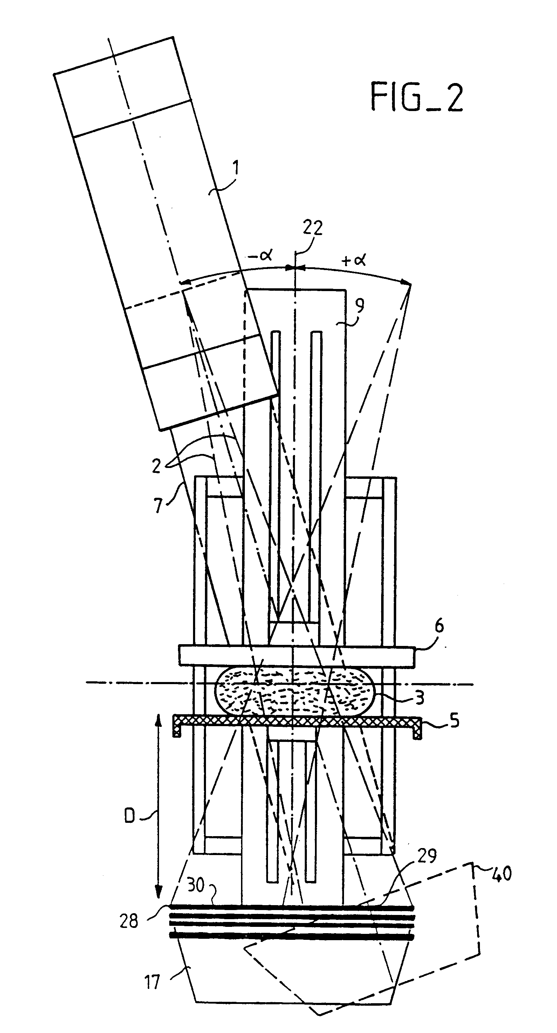

La figure 2 montre un mode d'utilisation du mammographe conformément à l'invention dans lequel on effectue des prises de vues stéréotaxiques du sein 3. Les images correspondant à ces prises de vues se reportent en juxtaposition sur un film 28 contenu dans la cassette 18. De part et d'autre de l'axe 22 du deuxième bras 9, et incliné avec des angles +ou -α, on a représenté des positions possibles en inclinaison du tube à rayons X 1 porté par le premier bras 7. On remarque premièrement que le récepteur d'images 17 est placé à une distance D du plateau porte-sein 5. Le rayonnement X peut être considéré comme limité par un limiteur 23 (figure 1) de telle façon que dans une première orientation (inclinée à gauche) l'image du sein 3 soit reportée sur la partie droite 29 du film 28. Dès que le cliché a été pris, il suffit de basculer le tube à rayons X 1 de l'autre côté de l'axe 22, de préférence d'un même angle α, pour que l'image du sein 3 soit alors reportée sur la partie gauche 30 du film 28. Entre-temps ce film a été maintenu dans sa position. On constate que l'effet de parallaxe, amplifié par l'agrandissement d'images imposé par l'écart D, permet d'aboutir au résultat suivant : les deux images sont juxtaposées et ne se confondent plus.

Par conséquent il est possible dans un dispositif de visualisation, ou dans un dispositif de traitement, d'utiliser les deux images ainsi juxtaposées pour conduire à une exploitation en relief du sein étudié. Dans la pratique on retient un angle α de l'ordre de 15° ce qui conduit à une distance D d'environ 25 cm. On peut néanmoins choisir des angles α plus grands et réduire en correspondance l'écart D. Pour permettre le maintien en position de l'arbre 9 et de tous les accessoires qu'il porte pendant cette double prise de vues stéréotaxiques l'arbre 11 peut être fixé solidairement a un bâti du mammographe par tous moyens. Dans le cas des prises de vues stéréotaxiques, le frein manoeuvré par la poignée 12 peut être relâché. Dans ce cas les bras sont manoeuvrés, depuis le bâti du mammographe, par des actions appliquées sur les arbres 10 et 11. Dans une première position incliné du tube à rayons X, le bras 7 est maintenu par rapport au mammographe dans une position prédéterminée, de préférence par des butées montées soit sur le bâti extérieur soit sur l'arbre 8. Le bâti du mammographe peut bien entendu comporter des butées correspondant à l'autre inclinaison, de l'autre côté de l'axe 22, du tube à rayons X. Ces butées peuvent être réglables et permettre les explorations symétriques (ou non) d'angles variables (15° à 30°).

Mais le mammographe de l'invention peut aussi être utilisé comme un mammographe de l'état de la technique. Dans ce but le frein manoeuvré par la poignée 12 peut être serré de telle façon que les deux bras 7 et 9 soient rendus solidaires. Lorsque ces deux bras sont verrouillés ensemble tout en étant de préférence parallèles, le bras mobile qu'ils constituent ensemble peut être utilisé pour des examens radiographiques de la même façon qu'un bras ordinaire. La figure 2 montre en tirets, dans le cas d'une telle solidarisation, la position correspondante 40 du récepteur d'images pour un cliché classique pris en incidence oblique -α. Dans cette incidence oblique -α, la représentation du plateau porte-sein et du plateau de compression n'est pas représentée. Elle devrait être inclinée par rapport aux dispositions représentées.

Il est possible que le limiteur 23 ne soit pas suffisant pour éviter un voile de la partie (30) du film correspondant à un autre cliché au moment de la prise d'un premier cliché (29). Dans ce but on peut prévoir le déplacement manuel, ou de préférence solidarisé au mouvement du bras 7 d'un masque de protection de la partie à ne pas impressionner du film 28. Par exemple, à proximité du contrepoids 24 du tube 1 sur le bras 7, on peut disposer deux baguettes coudées qui entraînent par leurs extrémités les bords d'un masque, plan ouvert dans sa partie centrale pour laisser passer le rayonnement, et qui est placé au-dessus du récepteur d'image 4. La position de l'ouverture du masque suit donc sensiblement la trace utile du rayonnement X. Au besoin on peut prévoir une motorisation et un asservissement électrique de la position du masque.

Le déplacement d'un masque présente, par rapport au déplacement des cassettes de l'état de la technique l'avantage que le film reste ici en place. Il est donc automatiquement réglé en position dans la cassette. Il en résulte que les calculs qui pourraient être faits, avec un même film doublement impressionné conformément à l'invention, seront entachés de moins d'erreurs que ceux qu'on pouvait effectuer en comparant deux clichés pris en déplaçant la cassette dans son logement. En effet, avec l'invention il n'y a pas à se préoccuper du calage de la position d'un cliché par rapport à l'autre. Quelle que soit la mise en place de la cassette 18 sur le porte cassette 17 les deux images se trouvent dans des positions cohérentes l'une par rapport à l'autre. Il en résulte une interprétation plus précise des vues stéréotaxiques. De ce fait un dispositif porte aiguilles de biopsie 25, comportant une aiguille 26, et fixé au plateau de compression 6, peut être approché avec une très grande précision d'une partie du sein 3 dans laquelle on veut effectuer un prélèvement. Une référence de position de ce porte-aiguille peut être obtenue très facilement en remplaçant l'aiguille par une petite bille (ou une croix) métallique judicieusement placée à un endroit connu sur le porte-aiguille. La trace de cette bille est visible sur chacun des clichés et donne une référence de position du porte-aiguille par rapport au sein radiographié.

Par sa maniabilité le mammographe de l'invention permet la suppression de la plus grande partie des dispositifs additionnels de l'état de la technique. Ces dispositifs étaient nécessaires aux prises de vues stéréotaxiques. En pratique le poids du dispositif additionnel constitué par le deuxième bras 9 est de l'ordre de trois ou quatre kilogrammes alors que le poids des dispositifs additionnels était d'environ quinze kilogrammes dans l'état de la technique. En effet il fallait aussi utiliser d'autres plateaux de compression 6, d'autres plateaux porte-sein 5 et d'autres plateaux porte-cassette 17, et d'autres dispositifs de compression.

L'invention se caractérise aussi par une utilisation astucieuse d'un mammographe. Dans cette utilisation, on éloigne d'une distance D le récepteur d'images du plateau porte-sein et on déplace, de part et d'autre de la normale le tube à rayons X seul en maintenant en place le récepteur d'images pour chaque cliché. De ce fait, les clichés obtenus bénéficient d'un grandissement favorable à la précision du dispositif de localisation.

## Revendications

1. Mammographe comportant des moyens pour maintenir en regard d'un sein (3) à radiographier un tube (1) à rayons X d'une part, et un plateau (5) support porte-sein et un récepteur (4) d'images radiographiques d'autre part, ces moyens de maintien comportant deux bras (7,9)
   – un premier bras (7) mobile en rotation autour d'un premier axe (8) perpendiculaire à ce premier bras, ce premier bras portant le tube à rayons X pour orienter (-$\alpha$,+$\alpha$) le rayonnement de ce tube,
   – un deuxième bras (9) portant le plateau support et le récepteur d'images, caractérisé en ce que le deuxième bras (9) est mobile en rotation autour d'un deuxième axe (8), ce deuxième axe étant perpendiculaire à ce deuxième bras, et dans un plan parallèle au plan de rotation du premier bras, et en ce que le deuxième bras (9) comporte des moyens (15,20) pour ménager un espace variable (D) entre le plateau porte-sein et le récepteur d'images, pour qu'une première image (29) du sein, projetée sur le récepteur d'images et correspondant à une première orientation du rayonnement du tube, soit juxtaposée sur ce récepteur d'images à une deuxième image (30) du même sein projetée dans les mêmes conditions mais correspondant à une deuxième orientation du rayonnement de ce tube, ces orientations différentes étant obtenues pour des positions différentes du premier bras (7) par rapport au deuxième bras (9).

2. Mammographe selon la revendication 1 caractérisé en ce que les deux axes de rotation des deux bras sont colinéaires et sensiblement horizontaux.

3. Mammographe selon l'une quelconque des revendications 1 ou 2 caractérisé en ce qu'il comporte des moyens (12) pour bloquer les mouvements en rotation relatifs d'un bras par rapport à l'autre dans une position quelconque.

4. Mammographe selon l'une quelconque des revendications 1 à 3 caractérisé en ce que le deuxième bras porte de plus un plateau (6) de compression du sein muni lui-même d'un porte-aiguille de biopsie.

5. Mammographe selon l'une quelconque des revendications 1 à 4 caractérisé en ce que le plateau porte-sein et le récepteur d'images radiographiques sont fixés au deuxième bras par des moyens (15,16) de coulissement de ces organes le long de ce bras.

6. Procédé de prise de vues stéréotaxiques d'un sein (3) avec un mammographe comportant les étapes suivantes :
   – on place un sein à radiographier entre un tube (1) à rayons X et un plateau (5) porte-sein,
   – on place un récepteur d'images (4) muni d'un film (28) radiosensible en aval du plateau porte-sein,
   – on écarte (D) ce récepteur d'image de ce plateau porte-sein, et on le rend solidaire (15,20) de ce plateau porte-sein,
   – on incline le tube d'un côté (-$\alpha$) de la normale (22) au plateau porte-sein, en maintenant immobile ce plateau porte-sein et ce récepteur d'images,
   – on prend un cliché (29),
   – on incline le tube d'un autre côté (+$\alpha$) par rapport à cette normale sans modifier ni la position du film radiosensible ni le récepteur d'images,
   – on prend un deuxième cliché (30),
   – et on développe l'image globale ainsi obtenue projetée en deux images juxtaposées sur un film unique radiosensible introduit dans le récepteur d'images.

## Claims

1. Mammography apparatus comprising means for positioning, opposite a breast (3) to be X-rayed, an X-ray tube (1) on the one hand and a breast supporting table (5) and an X-ray image receiver (4) on the other, these supporting devices comprising two arms (7,9):
   – a first arm (7) rotatable about a first axis (8) perpendicular to the first arm, the said first arm bearing the X-ray tube in order to orientate (- $\alpha$, + $\alpha$ ) the radiation of the said tube;
   – a second arm (9), bearing the support table and the image receiver, characterized in that the said second arm (9) is rotatable about a second axis (8), this second axis being perpendicular to the said second arm, and in a plane parallel to the plane of rotation of the first arm and that the second arm (9) comprises means (15,20) for providing a variable space (D) between the breast supporting table and the image receiver, so that a first image (29) of the breast, projected onto the image receiver and corresponding to a first orientation of the radiation of the tube, will be juxtaposed on this image receiver to a second image (30) of the same breast, projected under the same conditions but corresponding to a second orientation of the radiation of the said tube, these different orientations being obtained for different positions of the first arm (7) in relation to the second.

2. Mammographic apparatus in accordance with Claim 1, characterized by the fact that the two rotation axes of the two arms are co-linear and substantially horizontal.

3. Mammographic apparatus according to either of Claims 1,2, characterized in that it comprises means (12) for blocking relative movements of one arm in relation to the other in any desired position.

4. Mammographic apparatus according to any one of Claims 1-3, characterized in that the second arm also bears a breast compression plate (6) itself equipped with a biopsy needle holder.

5. Mammographic apparatus according to any of Claims 1-4, characterized in that the breast supporting plate and the X-ray image receiver are affixed to the second arm by means (15,16) for sliding these devices along the said arm.

6. Stereotaxic process for photographing a breast (3) with a mammographic apparatus, comprising the following stages:
   – a breast to be X-rayed is placed between an X-ray tube (1) and a breast supporting table (5);
   – an image receiver (4) equipped with a radio-sensitive film (28) is placed below the breast supporting table;
   – this image receiver is moved away (D) from this breast supporting plate and rendered integral (15,20) with the said breast supporting plate;
   – the tube is inclined (- α ) to one side of line (22) normal to the breast supporting plate, the said breast supporting plate and image receiver being kept motionless;
   – a photograph (29) is taken;
   – the tube is inclined towards the other side (+ α ) of the said normal line without altering either the position of the radio-sensitive film or that of the image receiver;
   – a second photograph (30) is taken;
   – and the total image is developed which has thus been obtained projected in the form of two juxtaposed images on one single radio-sensitive film introduced into the image receiver.

**Patentansprüche**

1. Mammographieapparat mit einerseits Einrichtungen zum Abstützen eine Röntgenstrahlröhre (1) gegenüber einer zu untersuchenden Brust (3) und andererseits einer der Abstützung der Brust dienenden Platte (5) sowie einem Aufnahmegerät (4) für die Röntgenbilder, wobei die Abstützeinrichtungen zwei Arme (7 und 9) umfassen,
   – wobei ein erster Arm (7) um eine erste senkrecht zu diesem ersten Arm stehende Achse (8) drehbeweglich angeordnet ist und wobei dieser erste Arm die Röntgenröhre trägt, um die Strahlen der Röhre auszurichten (-α und +α),
   – und wobei ein zweiter Arm (9) die Platte und das Aufnahmegerät für die Röntgenbilder trägt, dadurch gekennzeichnet, daß der zweite Arm (9) um eine zweite zu diesem zweiten Arm senkrecht stehende Achse (8), und in einer zu der Drehebene des Armes parallel liegenden Ebene drehbeweglich ist,
und daß der zweite Arm (9) Einrichtungen (15 und 20) umfaßt, um einen einstellbaren Raum (D) zwischen der Brustabstützplatte und dem Bildaufnahmegerät einzurichten, so daß ein erstes Bild (29) der Brust, das durch das Bildaufnahmegerät geworfen wird und das einer ersten Ausrichtung der Strahlen der Röhre entspricht, am Bildaufnahmegerät neben einem zweiten unter denselben Bedingungen geworfenen, dieselbe Brust darstellenden aber einer zweiten Ausrichtung der Strahlen der Röhre entsprechenden Bild (30) erzeugt wird, wobei diese verschiedenen Ausrichtungen in verschiedenen Stellungen des ersten Armes (7) relativ zum zweiten Arm (9) erhalten werden.

2. Mammographieapparat nach Anspruch 1, dadurch gekennzeichnet, daß die zwei Drehachsen der zwei Arme auf der gleichen Linie liegen und im wesentlichen horizontal sind.

3. Mammographieapparat nach Anspruch 1 oder nach Anspruch 2, dadurch gekennzeichnet, daß er Einrichtungen (12) umfaßt, um die Drehbewegungen eines Armes relativ zum anderen in einer beliebigen Stellung zu blockieren.

4. Mammographieapparat nach einem der vorhergehenden Ansprüche 1 bis 3, dadurch gekennzeichnet, daß der zweite Arm weiterhin eine mit einen Biopsiekanülenträger versehene Platte (6) zum Zusammendrücken der Brust umfaßt.

5. Mammographieapparat nach einem der vorhergehenden Ansprüche 1 bis 4, dadurch gekennzeichnet, daß die Brustabstützplatte und das Röntgenbildaufnahmegerät am zweiten Arm durch ein Gleiten dieser Teil entlang des Armes zulassende Einrichtungen (15 und 16) befestigt sind.

6. Verfahren zur Aufnahme von stereotaxische Ansichten einer Brust (3) unter Anwendung eines Mammographieapparats in folgenden Schritten:
   – Verbringen einer mittels Röntgenstrahlen abzubildenden Brust zwischen eine Röntgenstrahlröhre (1) und eine Brustabstützplatte (5),
   – Anordnen der mit einem für Röntgenstrahlen empfindlichen Film (28) versehenen Brustabstützplatte nach dem Röntgenbildaufnahmegerät (4),
   – Entfernen (D) des Röntgenbildaufnahmegeräts von der Brustabstützplatte und Anbringen (15 und 20) der Platte am Aufnahmegerät,

– Schwenken der Röhre zu einer Seite (-α) der Senkrechten (22) zur Brustabstützplatte und Halten der Brustabstützplatte und des Röntgenbildaufnahmegeräts in ihren Stellungen,

– Aufnehmen eines Bildes,

– Schwenken der Röhre zur anderen Seite (+α) relativ zur Senkrechten ohne die Stellung des für Röntgenstrahlen empfindlichen Filmes oder des Bildaufnahmegerätes zu verändern,

– Aufnehmen eines zweiten Bildes (30),

– und Entwickeln des so erhaltenen, in Form von zwei auf einem in das Bildaufnahmegerät eingeführten Film nebeneinander gelegenen projektierten Teilbildern vorliegenden Gesamtbildes.

FIG_1

FIG_2